# FASCICULE DE BREVET EUROPEEN

(11) **EP 2 032 966 B1**
(45) Date de publication et mention de la délivrance du brevet: **19.12.2012**
(21) Numéro de dépôt: 07788829.5
(22) Date de dépôt: 01.06.2007
(51) Int. Cl.: G01N 21/35, G01N 33/04

(54) **METHODE DE DETERMINATION DE LA QUALITE NUTRITIONNELLE DES LIPIDES DU LAIT**
VERFAHREN ZUR BESTIMMUNG DES NÄHRWERTS VON MILCHLIPIDEN
METHOD FOR DETERMINING THE NUTRITIONAL QUALITY OF MILK LIPIDS

(30) Priorité: 07.06.2006 FR 0605075
(43) Date de publication de la demande: 11.03.2009
(73) Titulaire: Valorex, 35210 Combourtille (FR)
(72) Inventeur: CHESNEAU, Guillaume, F-35210 Combourtille (FR)
(74) Mandataire: Regimbeau
(86) Numéro de dépôt international: PCT/FR2007/000917
(87) Numéro de publication internationale: WO 2007/141416

(56) Documents cités:
- WO-A-00/39578
- US-A- 5 252 829
- US-A- 5 343 044
- US-A1- 2005 250 212
- AZIZIAN H ET AL: "Quantification of trans fatty acids in food products by GC,ATR-FTIR and FT-NIR methods" LIPID TECHNOLOGY, BARKING ESSEX, GB, vol. 16, no. 10, octobre 2004 (2004-10), pages 229-231, XP003013510 ISSN: 0956-666X
- SOYEURT H ET AL: "Estimating fatty acid content in cow milk using mid-infrared spectrometry" JOURNAL OF DAIRY SCIENCE, vol. 89, no. 9, septembre 2006 (2006-09), pages 3690-3695, XP002455357 ISSN: 0022-0302

## Description

L'invention concerne une méthode de détermination de la qualité nutritionnelle des lipides du lait, dans laquelle on considère un nombre déterminé d'échantillons de lait de référence, on détermine, pour chacun de ces échantillons de référence, un profil d'acides gras et un spectre infrarouge obtenu par réflexion sur l'échantillon de référence d'un rayonnement dans l'infrarouge et on associe respectivement les profils d'acides gras aux spectres infrarouge, on soumet le lait à analyser, dont on veut déterminer la qualité nutritionnelle des lipides, à un rayonnement infrarouge pour, par réflexion, en déduire un spectre infrarouge et on compare le spectre infrarouge obtenu aux spectres infrarouge des échantillons de référence, pour en déduire un profil d'acide gras du lait à analyser.

La méthode repose donc sur l'appariement, ou l'association, d'un acide gras et d'une longueur d'onde dans l'infrarouge correspondant au rayonnement réfléchi par l'acide considéré et qui traduit donc son existence.

Les produits laitiers sont la première source quantitative de lipides dans les régimes de l'homme. Sous forme de beurre, fromage, boisson lactée et autres produits frais, les produits laitiers apportent, en France par exemple, plus de 30 g d'acides gras sur un total de 100 g par adulte et par jour.

Pourtant, la matière grasse laitière n'a pas une très bonne réputation en nutrition humaine et connaît depuis de nombreuses années un déclin de consommation. Les industriels laitiers recherchent continuellement des voies de valorisation et de différenciation qui les conduisent aujourd'hui à s'intéresser à la qualité de leur matière grasse.

A la différence des huiles végétales, qui contiennent une vingtaine d'acides gras majeurs différents, les matières grasses laitières sont composées d'un très grand nombre d'acides gras différents.

On connaît près de 400 acides gras différents dans les lipides du lait. Les proportions relatives de ces acides gras sont extrêmement variables en fonction de nombreux paramètres : la race des vaches, l'individu, la saison, le stade de lactation, le numéro de vêlage et surtout l'alimentation des vaches.

Les deux plus importants acides gras du lait sont un acide gras saturé: l'acide palmitique (C16:0) et un acide gras insaturé : l'acide oléique (C18 : 1 n-9). Ces deux acides gras représentent environ 50 % des acides gras du lait. Leurs proportions dans les acides gras totaux sont extrêmement variables. L'acide palmitique représente de 18 à 45 % des acides gras totaux. L'acide oléique représente de 12 à 35 % des acides gras totaux.

En dehors de ces deux acides gras, le lait contient également :
a. des acides gras saturés à chaîne courte (nombre d'atomes de carbone de la chaîne compris entre 2 et 10) ;
b. des acides gras saturés à chaîne moyenne : acide laurique (C12:0) et acide myristique (C14:0);
c. des acides gras monoènes cis et trans (principalement l'acide vaccénique C18:1 trans 11);
d. des acides gras conjugués (principalement le CLA cis9 trans 11);
e. des acides gras ramifiés ;
f. des acides gras polyinsaturés de la famille Oméga 3 (principalement l'acide alpha-linolénique C18:3 n-3);
g. des acides gras polyinsaturés de la famille Oméga 6 (principalement l'acide linoléique C18:2 n-6).

La grande variété et la grande dispersion de composition des acides gras du lait, d'une part, et l'importance quantitative de la consommation des lipides du lait, d'autre part, rendent très importants l'évaluation de la qualité nutritionnelle des lipides du lait.

Pour les nutritionnistes, il n'y a pas de bons et de mauvais acides gras, dans les régimes de l'homme, mais seulement des acides gras en excès et d'autres en déficit. Tous les guides nutritionnels s'accordent à recommander :
a. une hausse de la consommation d'acide oléique (C18: 1 n-9),
b. une hausse de la consommation d'acide alpha-linolénique(C18:3n-3),
c. une limitation de la consommation d'acide palmitique (C16:0),
d. une limitation de la consommation d'acide linoléique (C18:2n-6),
e. une augmentation du rapport C18 : ln-9 / C16:0,
f. une diminution du rapport C18 :2 n-6/C18 : 3 n-3.

Des effets intéressants seraient attribués également à la consommation d'acides gras conjugués (CLA cis 9 trans 11).

Il semble donc tout à fait intéressant de pouvoir évaluer de façon rapide et complète la qualité nutritionnelle des lipides du lait, à travers son profil en acides gras.

Cette évaluation est très difficile pour de nombreuses raisons :
a. nombre d'acides gras du lait à mesurer,
b. grande variation de la composition en acides gras (par exemple les valeurs mesurées pour l'acide alpha-linolénique Oméga 3 vont de 0,1 % à 2 % selon les laits),
c. difficultés et coût des techniques analytiques employées (chromatographie en phase gazeuse).

L'état de la technique en la matière est illustré par les documents US-A-2005/0250212, US-A-5 343 044 et US-A-5 252 829.

La présente invention a pour objet principal une méthode de détermination de la qualité nutritionnelle des lipides du lait, dans laquelle :
- on considère un nombre déterminé d'échantillons de lait de référence, et on détermine, pour chacun de ces échantillons de référence, un profil d'acides gras et un spectre infrarouge obtenu par réflexion sur l'échantillon de référence, d'un rayonnement dans l'infrarouge ;
- on associe respectivement les profils d'acides gras aux spectres infrarouges ;
- on soumet un lait à analyser, dont on veut déterminer la qualité nutritionnelle des lipides, à un rayonnement infrarouge pour, par réflexion, en déduire un spectre infrarouge et on compare le spectre infrarouge obtenu aux spectres infrarouges des échantillons de référence, pour en déduire un profil d'acide gras du lait à analyser,
   qui consiste à
- utiliser le rayonnement infrarouge dans la gamme de longueur d'onde 2500-10000 nm;
- à limiter la détermination du profil d'acides gras du lait à analyser par traitement infrarouge à certains acides gras de calibration satisfaisante ; et
- déduire par prédiction la teneur de certains autres acides gras à partir de celle des acides gras déterminée par traitement infrarouge.

Selon des caractéristiques avantageuses et non limitatives de ce procédé :
- on crée une base de données par détermination des spectres infrarouge d'un nombre élevé d'échantillons de lait de référence présentant un profil d'acides gras connu et déterminé par chromatographie en phase gazeuse (CPG).
- on a créé la base en procédant acide gras par acide gras.
- la base a été créée avec, pour chaque acide gras, une pluralité d'échantillons de référence à teneur déterminée par CPG.
- la base de données comporte une pluralité d'ensembles de quatre éléments comprenant un acide gras, sa teneur, la longueur d'onde et une puissance de calibration.

### Modèle de détermination de la composition en acides gras des lipides du lait

### a. Introduction

La détermination du profil en acides gras des lipides du lait s'effectue par la méthode de chromatographie en phase gazeuse (CPG). Elle permet de séparer des mélanges gazeux par suite d'équilibre entre une phase gazeuse mobile et une phase stationnaire. La méthode concerne des molécules volatiles naturellement, mais aussi des molécules qui sont rendue à des températures ne provoquant pas leur décomposition.

Chronologiquement, la méthode repose sur :
1 - une étape d'extraction de la matière grasse,
2 - une préparation des esters méthyliques d'acides gras,
3 - une analyse par chromatographie en phase gazeuse de ces esters méthyliques d'acides gras.

La durée et le coût de cette méthode de détermination la rendent difficilement opérationnelle pour les industriels laitiers en quête de valorisation de la matière grasse laitière par sa qualité nutritionnelle.

C'est ainsi qu'on a songé à une méthode tout aussi fiable, mais beaucoup plus rapide et moins onéreuse : l'analyse infrarouge.

### b. Matériel

Le matériel infrarouge à transformée de Fourier (I.R.T.F.) permet une définition spectrale dans l'infrarouge.

Le matériel I.R.T.F. opérant dans le proche infrarouge, à des longueurs d'onde de 1000 à 2500nm, peut être utilisé pour la détermination de profils d'acides gras de différentes huiles ou aliments solides. Il présente cependant deux inconvénients majeurs :
- la méthode est imprécise et manque de résolution, car les longueurs d'ondes, caractéristiques de différents acides gras se recoupent ;
- elle est mal adaptée à des produits liquides, pour lesquels une parfaite maîtrise de la température est nécessaire.

Au-delà de la chromatographie en phase gazeuse et de l'analyse dans le proche infrarouge, la demanderesse propose donc aujourd'hui une analyse dans l'infrarouge moyen, à des longueurs d'onde de 2500 à 10000 nm.

C'est une évolution audacieuse, dès lors que l'analyse infrarouge ne donnait pas satisfaction.

La méthode de détermination d'un mode de réalisation préféré, l'invention consiste à créer d'abord une base de données en déterminant les spectres infrarouge d'un nombre élevé d'échantillons de lait de référence présentant un profil d'acides gras connu et déterminé par la méthode de chromatographie en phase gazeuse.

En d'autre termes, dans un mode de réalisation préféré de l'invention, la demanderesse a considéré un grand nombre d'échantillons de lait de référence et procédé acide gras par acide gras.

Dans un mode réalisation préféré de l'invention pour chaque acide gras, elle a pris une pluralité d'échantillons de référence à teneur déterminée par CPG. Elle a bombardé ces échantillons d'un rayonnement IR moyen et obtenu par réflexion autant de spectres que d'échantillons de lait de référence en l'acide gras considéré. Ces spectres se recoupent en un point - ou une petite zone de longueurs d'onde - qui correspond à la longueur d'onde de l'acide gras considéré.

Procéder ainsi pour tous les acides gras choisis consiste à étalonner ou calibrer la méthode de détermination. Plus les zones de recoupement des spectres sont petites et assimilables à un point, plus la calibration est puissante ou précise.

Dans un mode de réalisation préféré de l'invention la base de données est donc constituée d'une pluralité d'ensembles de quatre éléments (acide gras, teneur, longueur d'onde, puissance de calibration). La teneur peut être le pourcentage moléculaire de l'acide gras considéré sur le total des acides gras.

Incidemment, la demanderesse a utilisé comme matériel IRTF l'appareil FT 6000 de la société Foss. Il est bien adapté au lait et présente une bonne stabilité.

La mesure est effectuée par analyse du signal de transmission infrarouge moyen au travers de l'échantillon situé dans une cellule de mesure. Le système optique retenu est constitué par un interféromètre de Michelson. Le signal global obtenu est ensuite décomposé par transformée de Fourier pour obtenir le spectre complet d'absorption de l'échantillon.

Ce spectre est lié directement à la composition chimique globale de l'échantillon et n'est donc pas spécifique de telle ou telle molécule, bien qu'il existe dans le domaine de l'infra-rouge moyen des zones d'absorption spécifiques des liaisons C-H, C=0, C-OH ou N-H caractéristiques de la chimie organique présente dans l'échantillon.

Le développement consiste donc à rechercher dans les spectres le poids des absorptions de chacune des longueurs d'onde permettant de définir l'acide gras étudié.

On conçoit que la qualité d'un calibrage infrarouge, pour analyser de nouveaux échantillons de lait, c'est-à-dire pour déterminer la qualité nutritionnelle de leurs lipides, dépend très fortement de la qualité de la base de données utilisée à cet effet. Celle-ci dépend du nombre et de la représentativité des échantillons qui la composent ainsi que de la qualité des valeurs obtenues pour les composés recherchés par la méthode de référence. En outre, cette banque de données doit recouvrir toute la gamme de mesure envisagée et les échantillons de référence doivent représenter toutes les matrices différentes qui pourront être soumises à l'essai.

La base de données mise en oeuvre par la demanderesse comprend près de 150 échantillons de lait de référence issus de zones d'élevage (ouest, est, nord et sud de la France), de troupeaux laitiers (races, génétique, niveau de production ...), de saisons (printemps, hiver) et de systèmes de rationnement (ration complète, semi-complète ...) et d'alimentation (ensilage de maïs, herbe pâturée, ensilage d'herbe, herbe enrubannée, foin, luzerne, concentrés énergétiques et protéiques, source d'apport lipidique) très variés, systèmes représentatifs de tous les modes de production et prenant en compte tous les facteurs de variation de la qualité des lipides du lait.

Ces échantillons de référence, traités dans l'infrarouge moyen, produisent des spectres caractéristiques dans une longueur d'onde donnée ou dans une bande suffisamment étroite pour être bien distinguée des autres.

La demanderesse, compte tenu des propos ci-dessus, a donc réalisé que la puissance, ou la précision, de la calibration variait d'un acide gras à un autre, qu'elle pouvait être satisfaisante pour l'un et pas pour un autre. Ainsi, pour l'acide palmitique C16:0, avec suffisamment d'échantillons, la précision de la calibration peut atteindre 90%, voire même la dépasser, mais pour les acides gras de la famille Omega 3, il n'en est rien.

C'est pourquoi, la demanderesse réalisant également que les proportions des divers acides gras étaient corrélées entre elles, a aussi songé à tirer profit de cette corrélation, pour définir des équations de prédiction en régression par exemple linéaire, et ainsi déterminer la teneur de certains acides gras de celle d'autres acides gras déterminés par traitement infrarouge avec une meilleure précision et donc à limiter la détermination du profil d'acide gras du lait à analyser par traitement infrarouge à certains acides gras de calibration satisfaisante.

Il est présenté ci-après un exemple de détermination de la composition d'un échantillon de lait en acides gras (AG) mineurs prédits statistiquement par régression à partir de la détermination en acides gras majeurs par traitement infrarouge.

La détermination est proposée à partir du tableau des équations de prédiction ci-dessous, dans lequel

| | | |
|---|---|---|
| AGS | représente | les acides gras saturés, |
| AGI | " | les acides gras insaturés, |
| AGPI | " | les acides gras polyinsaturés, |
| AGMI | " | les acides gras monoinsaturés et |
| r | " | la précision de résultat (coefficient de corrélation). |

### Equations de prédiction

| | |
|---|---|
| AGS + AGI = 100 | r=1,00 |
| AGPI + AGMI = AGI | r=1,00 |
| AGMI = 0,672 + 0,852 * AGI | r=0,98 |
| C14 :0=18,284-0,256*C18 :1 totaux | r=0,80 |
| C16 :0=35,35+0,231*AGS-0,587*AGI | r=0,87 |
| C18 :1 totaux=1,032*AGM1-5,157 | r=0,98 |
| C18 :1 *cis* totaux=0,76*C18 :1 totaux+0,646*AGMI-0,554*AGI+2,512 | r=0,98 |
| C18 :1 *cis*9=1,143*C18 :1 *cis* ttx-0,204*AGI-0,031*C18 :1 ttx+2,028 | r=0,99 |
| C18 :1 totaux= C18 :1 *cis* totaux + C18 :1 trans totaux | r=1,00 |
| C18 :3=0,005*AGI+0,193*AGPI-0,01*C16 :0-0,029 (Quand C16:0 > 25,5%) | r=0,86 |
| C18 :3=11,917 e^{(-0,1041C16 :0)} (Quand C16:0 < 25,5%) | r=0,83 |
| CLA cis9 trans11=0,366*AGPI-0,381*C18 :3-0,364 | r=0,78 |
| C18 :1 *trans* ttx=0,477*CLA cis9 trans11-0,043*AGS+1,362*C18 :1 *trans*11+3,428 | r=0,96 |
| C18 :2=0,657*AGPI-0,718*C18 :3-0,006*AGI-0,477*CLA cis9 trans11+0,292 | r=0,80 |
| C18 :1 *trans*11=2,319*CLA cis9 *trans*11+0.409*C18 :3-0,109*AGPI+0,034 | r=0,94 |
| C18 :1 *trans*10=0,438*C18 :1 *trans* ttx+0,091*CLA cis9 trans 11-0,556*C18 :1 *trans*11-0,114 | r=0,94 |

Les différents paramètres de prédiction du tableau ci-dessus entre les différents acides gras du lait ont été vérifiés et validés relativement aux mécanismes connus de synthèse des acides gras du lait dans le rumen et dans la mamelle des vaches.

### Exemple de détermination dé la qualité nutritionnelle des lipides du lait

1. Détermination par traitement infrarouge des acides gras saturés (AGS) du lait,
2. Détermination par différence des acides gras insaturés (AGI) du lait AGI = 100 - AGS
3. Détermination prédictive par régression linéaire des acides gras mono insaturés (AGMI) du lait :
   **AGMI=**O,672+0,852***AGI,** avec une précision de résultat r = 0,98
4. Détermination par différence des acides gras polyinsaturés (AGPI) AGPI = AGMI - AGI.
5. Détermination par traitement infrarouge du C16:0 et des C18 : 1 totaux du lait
**6-** Détermination prédictive par régression linéaire du C14 :0 du lait **C14 :0**=18,284-0,256*C18 :1 totaux r=0,80
**7-** Détermination prédictive par régression linéaire du C18 :3 n-3 du lait **C18 :3**=0,005***AGI**+0,193***AGPI**-0,01***C16** :0-0,029 r=0,86
**8-** Détermination prédictive par régression linéaire du CLA c9 t11 (ou CLA1) du lait **CLA1**=0,366***AGPI**-0,381***C18**:3-0,364 r=0,78
**9-** Détermination prédictive par régression linéaire du C18 :2 n-6 du lait **C18 :2**=0,657***AGPI**-0,718***C18 :3**-0,006*AGI-0,477*CLA1+0,292 r=0,82
**10-** Détermination prédictive par régression linéaire du C18 :0 du lait **C18 :0**=0,146*AGI-0,006*C18 :1 **totaux**+2,041***C18** :3+4,92 r=0,628
**11-** Détermination prédictive par régression linéaire du C18 :1 *t*11 du lait **C18 :1** *trans*11=2,319*CLA1+0,409*C18:3-0,109*AGPI+0,034 r=0,94
**12-** Détermination prédictive par régression linéaire du C18 :1 *cis* totaux du lait **C18 :1 *cis* totaux**=0,76*C18 :1 totaux+0,646***AGMI**-0,554***AGI**+2,512 r=0,98
**13-** Détermination prédictive par régression linéaire du C18 :1 cis9 du lait **C18 :1** *cis*9=1,143*C18 :1 *cis* ttx-0.204*AGI-0,031*C18 :1 ttx+2,028 r=0,99
**14-** Détermination prédictive par régression linéaire du C18 :1 *trans* totaux du lait **C18:1** *trans* ttx=0,477*CLA1-0,043*AGS+1,362*C18 :1 *trans*11+3,428 r=0,96
**15-** Détermination prédictive par régression linéaire du C18 :1 *trans*10 du lait **C18 :1** *trans*10=0,438*C18 :1 *trans* ttx+0,091*CLA1-0,556*C18 :1 *trans*11-0,114 r=0,94

Grâce à la technique d'analyse décrite ci-dessus, il est possible de connaître rapidement la qualité nutritionnelle des lipides du lait ci-dessus le jour même de sa production.

Les producteurs de lait et l'industrie laitière disposent ainsi d'un outil très fiable, très rapide et très pratique d'emploi qui va permettre d'accélérer le processus d'amélioration de la qualité nutritionnelle des lipides du lait.

## Revendications

1. Méthode de détermination de la qualité nutritionnelle des lipides du lait, dans laquelle :
- on considère un nombre déterminé d'échantillons de lait de référence, et on déterminer, pour chacun de ces échantillons de référence, un profil d'acides gras et un spectre infrarouge obtenu par réflexion sur l'échantillon de référence, d'un rayonnement dans l'infrarouge ;
- on associe respectivement les profils d'acides gras aux spectres infrarouges ;
- on soumet un lait à analyser, dont on veut déterminer la qualité nutritionnelle des lipides, à un rayonnement infrarouge pour, par réflexion, en déduire un spectre infrarouge et on compare le spectre infrarouge obtenu aux spectres infrarouges des échantillons de référence, pour en déduire un profil d'acide gras du lait à analyser,
qui consiste à
- utiliser le rayonnement infrarouge dans la gamme de longueur d'onde 2 500 - 10 000 nm;
- à limiter la détermination du profil d'acides gras du lait à analyser par traitement infrarouge à certains acides gras de calibration satisfaisante ; et
- déduire par prédiction la teneur de certains autres acides gras à partir de celle des acides gras déterminée par traitement infrarouge.

2. Méthode selon la revendication 1, **caractérisée par le fait qu'**on crée une base de données par détermination des spectres infrarouge d'un nombre élevé d'échantillons de lait de référence présentant un profil d'acides gras connu et déterminé par chromatographie en phase gazeuse.

3. Méthode selon la revendication 2, **caractérisée par le fait qu'**on a créé la base en procédant acide gras par acide gras.

4. Méthode selon la revendication 3, **caractérisée par le fait que** la base a été créée avec, pour chaque acide gras, une pluralité d'échantillons de référence à teneur déterminée par chromatographie en phase gazeuse.

5. Méthode selon l'une des revendications 2 à 4, **caractérisée par le fait que** la base de données comporte une pluralité d'ensembles de quatre éléments comprenant un acide gras, sa teneur, la longueur d'onde et une puissance de calibration.

## Claims

1. A method for determining the nutritional quality of milk lipids, involving the steps consisting of:
- considering a defined number of reference milk samples, determining, for each of said reference samples, a fatty acid profile and an infrared spectrum obtained through reflection on the reference sample of a radiation in the mean infrared;
- associating respectively the fatty acid profiles to the infrared spectra;
- subjecting the milk to be analyzed, the lipid nutritional quality of which is to be determined, to an infrared radiation, so as, through reflection, to infer an infrared spectrum; and comparing the milk infrared spectrum to be analyzed to the infrared spectra of the reference samples, so as to infer a fatty acid profile of the milk to be analyzed,
which consists in:
- making use of an infrared radiation in the wavelength range of 2 500 - 10 000 nm,
- limiting the determination of the milk fatty acid profile to be analyzed through infrared treatment to some fatty acids with a satisfactory reliability,
- obtaining the content of other fatty acids through prediction, from the content of said fatty acids as determined through infrared treatment.

2. A method according to claim 1, **characterized in that** a database is created through determination of the infrared spectra of a high number of reference milk samples showing a known fatty acid profile determined through gas phase chromatography.

3. A method according to claim 2, **characterized in that** the base is created working fatty acid by fatty acid.

4. A method according to claim 3, **characterized in that** the base was created with, for each fatty acid, a plurality of reference samples with a content to be determined via gas chromatography.

5. A method according to claim 2, **characterized in that** the database comprises a plurality of sets with four elements comprising a fatty acid, the content thereof, the wavelength and a calibration power.

## Patentansprüche

1. Verfahren zur Bestimmung des Nährwerts von Milchlipiden, bei dem:
- man eine bestimmte Zahl von Referenz-Milchproben betrachtet und für jede der Referenz-Proben ein Fettsäuren-Profil und ein Infrarotspektrum bestimmt, das mittels Reflexion einer Strahlung im Infrarot an der Referenz-Probe erhalten wird;
- man jeweils die Fettsäuren-Profile mit den Infrarotspektren in Beziehung bringt;
- man eine zu analysierende Milch, bei der man den Nährwert der Lipide bestimmen will, einer Infrarotstrahlung aussetzt, um mittels Reflexion daraus ein Infrarotspektrum herzuleiten, und man das erhaltene Infrarotspektrum mit den Infrarotspektren der Referenz-Proben vergleicht, um daraus ein Fettsäure-Profil der zu analysierenden Milch abzuleiten,
welches darin besteht,
- die Infrarotstrahlung in einem Wellenlängenbereich von 2.500 - 10.000 nm zu verwenden;
- die Bestimmung des Fettsäuren-Profils der zu analysierenden Milch durch Infrarotbehandlung auf gewisse Fettsäuren mit zufriedenstellender Kalibrierung zu begrenzen und
- durch Vorhersage den Gehalt gewisser anderer Fettsäuren ausgehend von denjenigen Fettsäuren, die durch Infrarotbehandlung bestimmt worden sind, abzuleiten.

2. Verfahren nach Anspruch 1, **dadurch gekennzeichnet, dass** man eine Basis von bekannten Größen durch Bestimmung der Infrarotspektren einer hohen Zahl von Referenz-Milchproben schafft, die ein bekanntes und durch Gaschromatographie bestimmtes Fettsäuren-Profil aufweisen.

3. Verfahren nach Anspruch 2, **dadurch gekennzeichnet, dass** man die Basis schafft, indem man Fettsäure für Fettsäure verfährt.

4. Verfahren nach Anspruch 3, **dadurch gekennzeichnet, dass** die Basis für jede Fettsäure mit einer Mehrzahl von Referenz-Proben mit einem durch Gaschromatographie bestimmten Gehalt geschaffen worden ist.

5. Verfahren nach einem der Ansprüche 2 bis 4, **dadurch gekennzeichnet, dass** die Basis von bekannten Größen eine Mehrzahl von Einheiten von vier Elementen umfasst, welche eine Fettsäure, deren Gehalt, die Wellenlänge und ein Kalibrierungsvermögen umfassen.
